# EUROPÄISCHE PATENTSCHRIFT

(11) **EP 0 873 362 B1**
(45) Veröffentlichungstag und Bekanntmachung des Hinweises auf die Patenterteilung: **12.06.2002**
(21) Anmeldenummer: 97900978.4
(22) Anmeldetag: 09.01.1997
(51) Int. Cl.: C07K 16/06, C07K 1/14, A61K 39/395, A61L 2/00

(54) **VERFAHREN ZUR TRENNUNG VON IgG und IgA**
PROCESS FOR ISOLATING IgG AND IgA
PROCEDE D'ISOLATION DES IMMUNOGLOBULINES G et A

(30) Priorität: 12.01.1996 DE 19600939
(43) Veröffentlichungstag der Anmeldung: 28.10.1998
(73) Patentinhaber: Baxter Aktiengesellschaft, 1221 Wien (AT)
(72) Erfinder: LEIBL, Heinz, A-1120 Wien (AT); TOMASITS, Regine, A-1190 Wien (AT); MANNHALTER, Josef, A-1050 Wien (AT); WOLF, Hermann, A-3411 Weidling (AT); EIBL, Martha, A-1180 Wien (AT)
(74) Vertreter: Kolb, Helga, Dr. Dipl.-Chem.
(86) Internationale Anmeldenummer: EP9700067
(87) Internationale Veröffentlichungsnummer: WO9725352

(56) Entgegenhaltungen:
- EP-A- 0 692 491
- DE-A- 2 404 265
- JP-A- 3 291 300
- US-A- 4 745 183
- US-A- 5 258 177
- INTERNATIONAL BIOTECHNOLOGY LABORATORY, Bd. 3, Nr. 4, 1985, Seiten 33-42, XP002029969 STEVENS AND BROOKS: "Techniques for purifying monoclonal antibodies"
- PROTEIN EXPRESSION AND PURIFICATION, Bd. 6, 1995, Seiten 408-410, XP002029970 LEIBL ET AL.: "Isolation of human serum IgA using thiophilic adsorption chromatography"
- JOURNAL OF CHROMATOGRAPHY, Bd. 678, Nr. 2, 12.April 1996, Seiten 173-180, XP000673220 LEIBL ET AL.: "Method for the isolation of biologically active monomeric immunoglobulin A from a plasma fraction"

## Beschreibung

Die Erfindung betrifft ein Verfahren zur Trennung von IgG und IgA sowie die gemäss diesem Verfahren erhältliche IgA-Lösungen.

Immunglobuline (Ig) sind spezifische Abwehrproteine im Blutplasma, der Lymphe und in anderen Körpersekreten aller Wirbeltiere. Die Immunglobuline werden von B-Lymphocyten synthetisiert. Die monomeren Immunglobuline bestehen aus je zwei L (light)- und H (heavy)-Ketten, die durch Disulfidbrücken miteinander verbunden sind. Die Immunglobuline sind Glycoproteine, die als Antikörper fungieren, und deren Bildung durch Antigene stimuliert wird. Mengenmässig machen sie ca. 20% des gesamten Plasma-Proteins aus.

Von den Immungobulinen wurden beim Menschen bisher 5 Hauptklassen identifiziert (IgA, IgD, IgE, IgG und IgM), die sich in ihren H-Ketten, in ihrer Serum-Konzentration, dem Molekulargewicht (ca. 146000 bis 970000), dem Kohlenhydratgehalt, der elektrophoretischen Beweglichkeit und in ihren biologischen Eigenschaften unterscheiden. Die Hauptklassen IgA und IgG lassen sich noch in Subklassen unterteilen (z.B. IgA1, IgA2). Die Vielfalt der Immunglobulin-Klassen und -Unterklassen sowie deren vielfältige unterschiedliche Spezifität in der Bindung von Antigenen kommt durch Kombination verschiedener genetischer Bauteile zustande.

Immunglobulin A (IgA) stellt die Hauptantikörperklasse in äusseren Sekreten, wie Speichel, Tränenflüssigkeit und dem Schleim des Bronchial- und des Intestinaltraktes dar. Somit bildet Immunglobulin A eine der ersten Verteidigungslinien gegen bakterielle und virale Pathogene.

In der reinen monomeren Form besteht IgA aus zwei leichten (L) und zwei schweren (H) Ketten; in der dimeren sekretorischen Form sind zwei solche Monomere durch die sogenannte J-Kette (joining chain) gekoppelt. In den Sekreten der Schleimhäute und Drüsen kommen vor allem Dimere mit einer zusätzlichen sekretorischen Komponente (sogenannte SC-Komponente) vor.

In Lösung liegt plasmatisches IgA-Monomer im Gleichgewicht mit nicht kovalent gebundenem IgA-Dimer vor. In diesem Gleichgewicht beträgt der Anteil an dimerem IgA maximal etwa 25% des Gesamt-IgA.

IgA besteht aus zwei Subklassen, IgA1 und IgA2, die in einem nativen Verhältnis von etwas 80 Gew.-% zu 20 Gew.-% vorliegen. Dieses Verhältnis kann im Rahmen einer Isolierung verändert werden. Das native Verhältnis von kappa- zu lambda-Leichtketten (gemessen in U/dl) beträgt in einem Immunglobulinpräparat etwa 1:1.

IgA stellt nur ca. 3-4% des Gesamtproteins von normalem humanem Serum dar. Während der Reinigung von IgA wurde eine ausgeprägte Tendenz zur Komplexbildung und Aggregation beobachtet. Die Isolierung des monomeren IgA aus Serum war daher meist mit geringen Ausbeuten verbunden, und unter den zahlreichen Herstellungsverfahren sind bislang nur wenige bekannt, die auch für eine grosstechnische Produktion geeignet sind. Hauptverunreinigungen der IgA-Präpationen sind im wesentlichen die verschiedenen Unterklassen von Immunglobulin G, deren Abtrennung zusätzliche, die Ausbeute an IgA weiter vermindernde Reinigungsschritte erfordern.

Die bekannten und gängigen Methoden zur Reinigung von Immunglobulinen beruhen zumeist auf Unterschieden in den physikalischen Eigenschaften, wie z.B. Löslichkeit in wässrigen Systemen (Reinigung durch fraktionierte Fällung), Anteil an Ladungen (Reinigung durch Ionenaustauschchromatographie) oder Unterschiede in der Molekülgrösse (Reinigung durch Molekularausschlusschromatographie).

Zur Durchführung chromatographischer Reinigungsmethoden werden seit langem auch anorganische Trägermaterialien, beispielsweise für die Auftrennung von Plasmaproteinen verwendet. So wird z.B. Al(OH)₃ zur Trennung des Prothrombinkomplexes von anderen im Plasma enthaltenen Proteinen eingesetzt, und dieses Verfahren wird auch in grosstechnischem Maßstab angewandt. Auch andere anorganische Materialien, wie z.B. Aluminiumoxide, Calciumphosphate oder unterschiedlichste Verbindungen des Silicium, werden bevorzugt bei der Präparation im grosstechnischen Maßstab eingesetzt.

Aus EP-242 544 ist bekannt, Hydroxylapatit, welcher nach einem speziellen Herstellungsverfahren in gesinteter Form erhalten wird, als Adsorptionsmittel in der allgemeinen Chromatographie einzusetzen. Hydroxylapatit wird als chemische Substanz mit der Formel Ca₁₀(PO₄)₆(OH)₂, also als ein hydroxyliertes Calciumphosphat, beschrieben, welches zumeist in partikulärer Form vorliegt. Hydroxylapatit kann somit als eine besondere Form des Calciumphosphats betrachtet werden. U.a. wird Hydroxylapatit als ein geeignetes Material für die Trennung biologischer Makromoleküle, beispielsweise von Proteinen, wie Immunglobuline oder Enzyme, oder für die Trennung von RNA, DNA, Viren oder Plasmiden vorgeschlagen.

Aus DE-39 27 112 ist ein Verfahren zur Herstellung eines intravenös verabreichbaren Immunglobulin-haltigen Arzneimittels bekannt. Dieses Arzneimittel besteht aus IgA, IgG und IgM in ankonzentrierter Form und wird durch ein mehrstufiges Reinigungsverfahren, welches u.a. auch eine Absorption an Calciumphosphat in Gegenwart von Oktansäure beinhaltet, erhalten. Bei diesem Verfahren werden die Immunglobuline jedoch nicht voneinander getrennt, sondern es wird ein Arzneimittel bereitgestellt, das alle drei Gruppen von Immunglobulinen in einem bestimmten Mischungsverhältnis enthält.

Ausserdem wurden zur Prophylaxe und Behandlung bakterieller und viraler Infektionen bereits pharmazeutische Zusammensetzungen auf der Basis von Immunglobulinen, z.B. von Immunglobulin A, vorgeschlagen (vgl. z.B. JP-A-478 59815).

EP-692 491, das Stand der Technik nach Artikel 54(3) bildet, offenbart ein Verfahren zur Herstellung von virussicheren, monomeren IgA. Das Reinigungsverfahren stützt sich auf die Anwendung einer Gelpermeationschromatographie und/oder einer thiophilen Chromatographie, wobei als weiterer Reinigungsschritt eine Affinitätschromatographie durchgeführt werden kann. Das IgA-enthaltende Ausgangsmaterial kann auch vorgereinigt sein, beispielsweise durch eine Fällung mit Ethanol, Polyethylenglykol und/oder Ammoniumsulfat, und/oder durch ein weiteres chromatographisches Verfahren, z.B. unter Einsatz eines Ionenaustauschers, einer Adsorption an Hydroxylapatit, und/oder Dextransulfat und/oder eine Reinigung an Heparin-Sepharose. Die Vorreinigung, bei der ein Hydroxylapatitsäule angewendet wird, wird unter Bedingungen durchgeführt, unter denen IgA an Hydroxylapatit bindet.

JP-A-3291300 beschreibt die Auftrennung von Immunglobulinen mit Hilfe von Hydroxylapatit. Die Immunglobuline IgA, IgG und IgM werden dabei auf eine Hydroxylapatitsäule aufgebracht, adsorbiert und mittels einen Ionenstärkegradienten selektiv desorbiert. Es wird nicht erwähnt, eine Adsorption von IgA an das Säulenmaterial zu vermeiden, um auf diese Weise zu einer IgA-Lösung mit geringer Tendenz zur Aggregatbildung zu gelangen.

Ein Verfahren zur Gewinnung monoklonaler Antikörper unter Verwendung einer HPLC-Chromatographie in Kombination mit Hydroxylapatit wurde beschrieben (Int. Biotechnol.Lab.3/4 1985, S. 33-42), nicht aber ein Trennungsverfahren zur Gewinnung in IgA.

Ein Problem, das sich speziell bei der Verwendung von humanem Ausgangsmaterial für die Herstellung von Immunglobulinen stellt, ist die Virussicherheit des erhaltenen Produktes. Trotz Spenderauswahl und Testung der Einzelspenderplasmen ist nicht auszuschliessen, dass z.B. aufgrund der geringen Empfindlichkeit mancher Tests noch infektiöse Krankheitserreger, insbesondere Hepatitisviren oder Retroviren, wie HIV, im Spenderpool vorhanden sind.

Obwohl bei der Herstellung einer Immunglobulinpräparation durch fraktionierte Alkoholfällung nach Cohn eine Abreicherung/Inaktivierung von Viren um mehr als 10¹⁵ Einheiten beschrieben wurde (vgl. z.B. Wells et al, Transfusion 26 (1986) 120-213), besteht gerade bei Verwendung von Zwischenprodukten aus der Cohn-Fraktionierung immer noch die Gefahr einer unzureichenden Virusinaktivierung, d.h. einer nicht ausreichenden Virussicherheit.

Bei den üblichen Verfahren zur Inaktivierung von Viren muss mit einer zusätzlichen Aggregatbildung der Immunglobuline gerechnet werden. Dies zeigt sich in besonderem Masse bei den Verfahren der Hitzebehandlung, die bevorzugt angewendet werden, da neben lipidumhüllten Viren auch nicht-lipidumhüllte Viren (z.B. Hepatitis A-Viren) effektiv inaktiviert werden.

Es hat sich gezeigt, dass bei der vorstehend genannten Hitzebehandlung ein wesentlicher Anteil an IgA multimerisiert bzw. polymerisiert werden kann. Derart aggregiertes IgA verringert aber - wie vorstehend ausgeführt - die mit Hilfe verschiedener Reinigungsverfahren erzielten Ausbeuten an monomerem IgA. Aggregate, beispielsweise IgG-Aggregate, verursachen u.a. auch eine Erhöhung der antikomplementären Aktivität und führen damit zu Unverträglichkeitsreaktionen nach intravenöser Verabreichung. Deshalb wird nach dem Stand der Technik bei einigen Reinigungsverfahren, in denen ein Schritt zur Inaktivierung der Viren, und insbesondere eine Hitzebehandlung durchgeführt wird, in Gegenwart von Stabilisatoren gearbeitet. Ein derartiges Verfahren wird z.B. in EP-177 836 beschrieben. Ein Nachteil der Verwendung von Stabilisatoren besteht aber darin, dass dies einen weiteren Schritt zur Abtrennung derselben erfordert. Ein weiterer Nachteil der Stabilisatoren ist auch die gleichzeitige Stabilisierung von viralem Protein und damit die Verschlechterung der Kinetik zur Inaktivierung von Viren.

Aufgabe der vorliegenden Erfindung ist es, mittels eines einfach durchzuführenden und leicht in den grosstechnischen Maßstab übertragbaren Verfahrens, die Immunglobuline IgG und IgA in einem Immunglobulin-haltigen Ausgangsmaterial sowohl voneinander als auch von ihren hochmolekularen Aggregaten sowie anderen hochmolekularen kontaminierenden Substanzen, die entweder im Ausgangsmaterial bereits vorhanden waren oder die im Rahmen der Reinigungsschritte gebildet wurden, zu trennen.

Die vorstehende Aufgabe wird gemäss der Erfindung durch ein Verfahren gelöst, welches dadurch gekennzeichnet ist, dass
(i) IgG unter Bedingungen an das Trägermaterial Hydroxylapatit adsorbiert wird, unter denen IgA nicht daran adsorbiert wird,
(ii) IgA das nicht an das Trägermaterial Hydroxylapatit adsorbiert wird, gewonnen wird, während IgG am Trägermaterial verbleibt

Unter der Bezeichnung 'Eluat' wird gemäss der Erfindung eine wässrige Lösung verstanden, welche durch Behandlung des Ausgangsmaterials mit dem erfindungsgemäss verwendeten Trägermaterial erhalten wird. Die Bezeichnung 'Adsorbat', aus welchem erfindungsgemäss IgG gegebenenfalls gewonnen wird, bedeutet dass festes Trägermaterial an dem IgG adsorbiert ist. Durch Desorbieren von IgG vom Trägermaterial kann IgG aus dem Adsorbat gewonnen werden.

Erfindungsgemäss wird als Immunglobulin-haltiges Ausgangsmaterial Plasma, Serum oder eine entsprechende Plasmafraktion eingesetzt. Im weiteren kann es sich bei dem Ausgangsmaterial auch um Sekretionen, z.B. mukosale Sekrete, handeln. Vorzugsweise wird ein humanes Ausgangsmaterial verwendet. Besonders bevorzugt werden Plasma und/oder Plasmafraktionen verwendet, welche gegebenenfalls zunächst nach der Methode von Cohn mit Ethanol behandelt, und es wird dann die Cohn II+III-Fraktion als Ausgangsmaterial für das erfindungsgemässe Verfahren eingesetzt.

Das anorganische Trägermaterial ist Hydroxylapatit, insbesondere keramisches Hydroxylapatit.

Die Bedingungen für die Adsorption an das anorganische Trägermaterial werden so gewählt werden, dass IgG adsorbiert wird. Vorzugsweise werden auch hochmolekulare Proteine und/oder Proteinaggregate an das Trägermaterial adsorbiert. Bei letzteren hochmolekularen Substanzen kann es sich um solche handeln, die bereits ursprünglich im Ausgangsmaterial vorliegen oder um Aggregate, die erst während des Reinigungsverfahrens gebildet werden. Derartige Proteinaggregate können auch Immunglobulinaggregate darstellen, wie beispielsweise IgG- oder IgA-Aggregate. Immunglobuline zeigen im allgemeinen bei der Behandlung mit chemischen Substanzen oder auch bei Anwendung erhöhter Temperaturen etc eine starke Tendenz, sich zu aggregieren. Dies ist besonders bei einer Hitzebehandlung zur Inaktivierung von Viren oder bei den Schritten der Plasmafraktionierung der Fall. Das Vorhandensein von aggregierten Immunglobulinen ist aber gerade bei der Herstellung intravenös anzuwendender pharmazeutischer Präparationen problematisch.

Durch geringfügige Erhöhung der Ionenstärke im Puffer kann überraschenderweise eine selektive Adsorption des IgG gegenüber IgA erreicht werden. Das IgA unterliegt dabei keiner bzw. einer geringen Adsorption an das Trägermaterial und wird daher keinem bzw. einem wesentlich geringeren physikalischen Stress ausgesetzt, als dies beispeilsweise im Falle einer Adsorption der Fall ist. Auf diese Art und Weise wird gemäss der Erfindung die Bildung hochmolekularer IgA-Aggregate vermieden bzw. die Tendenz zur Aggregatbildung verringert.

Ein Puffer mit einem pH-Wert zwischen 6,5 und 7,5 und einer Ionenstärke entsprechend einer Konzentration an Phosphat zwischen 0 bis 10 mM bindet IgA quantitativ an das feste Trägermaterial. Eine selektive Desorption des IgA, während IgG am Trägermaterial verbleibt, erfolgt dann bei Verwendung eines Puffers, dessen Ionenstärke entsprechend einer Konzentration an Phosphat zwischen 11 und 100 mM vorzugsweise zwischen 20 und 40 mM, liegt. Erfindungsgemäß wird der letztgenannte Puffer für die selektive Adsorption des IgG verwendet werden, während IgA im Eluat verbleibt.

Der Puffer zur Durchführung des erfindungsgemässen Verfahrens enthält bevorzugterweise keine höheren organischen Säuren, so enthält der Puffer beispielsweise keine Oktansäure, die u.U. Protein-fällend wirkt.

Weitere Reinigungsschritte können dem erfindungsgemässen Verfahren vor- oder nachgeschaltet sein. Hierfür kommen alle üblichen Methoden, wie z.B. chromatographische Verfahren, Fällungen und/oder weitere Adsorptionsschritte infrage. Das chromatographische Verfahren kann eine Ionenaustausch-, Affinitäts-, thiophile und/oder eine Gelpermeationschromatographie sein. Die Fällungen können beispielsweise mit ZnSO₄, Rivanol, Ethanol, Polyethylenglykol und/oder Ammoniumsulfat, auch als fraktionierte Fällungen durchgeführt, erfolgen. Als Adsorptionsmaterialien kommen neben weiteren anorganischen Stoffen auch organische Stoffe, wie Agarose, Sepharose oder PEG-Derivate, infrage. Weitere Reinigungsschritte können diverse Filtrations- und Zentrifugationsschritte umfassen.

Bevorzugterweise wird als zusätzliches Reinigungsverfahren eine Ionenaustauschchromatographie durchgeführt. Am meisten bevorzugt erfolgt zusätzlich noch eine Fällung, beispielsweise mit Ammoniumsulfat. Gegebenenfalls wird als ein weiterer Schritt noch eine Adsorption an Protein-G-Sepharose durchgeführt zur selektiven Adsorption von IgG.

Alle Reinigungsschritte unter Verwendung eines Trägermaterials können im Batch-Verfahren oder in einer Säule durchgeführt werden. Gerade für den grosstechnischen Einsatz ist das Batch-Verfahren vorzuziehen, da damit im allgemeinen grössere Mengen an Ausgangsamterial in kürzerer Zeit gereinigt bzw. aufgetrennt werden können.

Nach einer bevorzugten Ausführungsform wird auch ein Schritt zur Inaktivierung von Viren durchgeführt. Dieser Schritt kann an beliebiger Stelle des erfindungsgemässen Reinigungsverfahrens bzw. der weiteren verschiedenen Reinigungsschritte erfolgen. Vorzugsweise wird ein Schritt zur Inaktivierung von Viren nach der Behandlung mit einem festen anorganischen Trägermaterial durchgeführt.

Zur Virusinaktivierung können die im Stand der Technik bekannten Verfahren angewendet werden. Dabei ist bekannt, dass bevorzugt eine Hitzebehandlung erfolgt, da diese auch eine Inaktivierung von nicht-lipidumhüllten Viren (z.B. Hepatitis A) bewirkt.

Zur Durchführung einer Hitzebehandlung kann die Präparation in gelöster oder fester Form eingesetzt werden. Bei einer Hitzebehandlung in fester Form besitzt das der Hitzebehandlung zu unterziehende Produkt vorzugsweise einen Feuchtigkeitsgehalt von 5 bis 70 Gew.-%. Nach einer anderen bevorzugten Ausführungsform wird als feste Form ein Lyophilisat eingesetzt. Die Virusinaktivierung erfolgt vorzugsweise bei einer Temperatur im Bereich zwischen 40 und 80°C, insbesondere im Bereich zwischen 50 und 65°C. Die Hitzebehandlung wird dabei mindestens zu einem für die Inaktivierung von Viren ausreichend langem Zeitraum durchgeführt, vorzugsweise während einer Dauer von 30 Minuten bis 10 h. Bevorzugt erfolgt die Hitzebehandlung als Dampfbehandlung, insbesondere nach einem in EP-159 311 beschriebenen Verfahren. Bei dem genannten Verfahren, welches ohne Zusatz von Stabilisatoren arbeitet, können anstelle von Wasser auch Hydroxylgruppen-haltige Verbindungen, wie z.B. Methanol, Ethanol oder Mannit verwendet werden.

Es ist auch möglich, die Virusinaktivierung in Gegenwart von Stabilisatoren durchzuführen. Ein solches Verfahren wird in EP-177 833 beschrieben. In diesem Verfahren schützt der Stabilisator die relativ labilen Immunglobuline vor Denaturierung und erhält somit deren biologische Aktivität.

Ausser einer Hitzebehandlung können aber auch alle anderen Verfahren zur Inaktivierung von Viren verwendet werden. So kann beispielsweise eine Solvent/Detergenz-Behandlung nach EP-131 740 oder eine Detergenzbehandlung nach EP-50 061 angewendet werden. Auch Methoden, die eine UV-Bestrahlung zusammen mit β-Propiolacton als Verfahren zur Inaktivierung von Viren beinhalten, sind geeignet.

Als weitere Verfahren, Immunglobulin-haltige Fraktionen einem Verfahren zur Inaktivierung von infektiösen Agenzien zu unterwerfen, sind chemische oder physikalische Behandlungen in Gegenwart eines Polyethers zielführend, wie dies in DE-44 34 538 beschrieben ist. Als Polyether kommen zu diesem Zwecke Polyhydroxyether, wie beispielsweise Polyalkylenglykol, und insbesondere Polyethylen- oder Polypropylenglykol infrage.

Als weitere Möglichkeit zur Inaktivierung vermehrungsfähiger Krankheitserreger ist auch die Verwendung neutraler Peptidhydrolasen, wie beispielsweise Trypsin oder Chymotrypsin, gemäss EP-247 998 zu erwähnen.

Es kann auch zweckmässig sein, die Hitzebehandlung mit einem oder mehreren anderen zur Vireninaktivierung bekannten und üblichen Verfahren zu kombinieren, insbesondere mit einer UV-Bestrahlung, einer Behandlung mit Tensiden und/oder einer Behandlung mit einem Solvens/Detergenz-System. Die einzelnen Verfahrensschritte können dabei gleichzeitig (z.B. Hitzebehandlung unter gleichzeitiger UV-Bestrahlung) oder in beliebiger Reihenfolge hintereinander erfolgen.

Durch eine Kombination der Hitzebehandlung mit einer oder mehreren anderen Verfahren zur Vireninaktivierung lassen sich die unterschiedlichen Wirkungsmechanismen dieser Inaktivierungsverfahren ausnutzen, wodurch die Virussicherheit des Produktes noch weiter erhöht werden kann.

Eine bewährte Massnahme zur Abreicherung von Viren ist auch die Nanofiltration einer die Immunglobuline enthaltenden Lösung.

Gegebenenfalls wird auch das IgG aus dem Adsorbat gewonnen, wobei das IgG nach Desorption von dem festen, anorganischen Trägermaterial zur Verfügung gestellt wird. Das IgG kann aber auch durch weitere Verfahren gereinigt werden, wie dies bereits für die Gewinnung von Immunglobulinen an früherer Stelle beschrieben wurde. Die weiteren Reinigungsverfahren für das IgG entsprechen den aus dem Stand der Technik bekannten Techniken (siehe beispielsweise AT-0383 737, Immuno AG, oder J J Langone in J Immunol Methods 55 (1982), S. 277-296).

Mittels des erfindungsgemässen Verfahrens kann somit lediglich IgA gewonnen werden, man kann aber auch zusätzlich IgG-Präparationen erhalten.

Vor der Hitzebehandlung kann zweckmässigerweise auch eine Dialyse gegen Wasser durchgeführt werden, wodurch sich gegebenenfalls noch vorhandene Verunreinigungen, insbesondere gegebenenfalls eingesetzte oder vorhandene stabilisierende Substanzen, weitgehend entfernen lassen.

Durch das erfindungsgemässe Verfahren wird ein IgA-Präparat erhalten, welches im wesentlich frei von IgG ist. Unter dem Begriff 'IgA im wesentlichen frei von IgG' ist erfindungsgemäss ein IgA zu verstehen, welches weniger als 15% IgG enthält, vorzugsweise 10% und am meisten bevorzugt 2 bis 5% IgG, bezogen auf Gesamtimmunglobulin, umfasst.

Die Reinheit des erfindungsgemässen IgA-Präparates liegt über 70% und beträgt vorzugsweise 80 bzw. 85%, wobei am meisten bevorzugt ein erfindungsgemässes IgA-Präparat von 90 bis 92% Reinheit, bezogen auf Gesamtprotein, ist.

Wie bereits vorstehend erwähnt werden die Bedingungen für die Adsorption an das anorganische Trägermaterial Hydroxylapatit derart gewählt, dass IgG absorbiert, während IgA nicht adsorbiert wird und aus der als Eluat bezeichneten wässrigen Lösung bzw. Suspension gewonnen wird. IgA wird während dieses Reinigungsschrittes keinem physikalischen Stress ausgesetzt. Dadurch werden Veränderungen im Molekül, beispielsweise Konformationsänderungen, wie sie gerade aufgrund der Adsorption auftreten können, grösstenteils vermieden. Erfindungsgemäss ist es bevorzugt, auch bei weiteren Reinigungsschritten die Bedingungen derart zu wählen, dass eine Adsorption des IgA vermieden wird. Dies ist insofern von Bedeutung, da physikalischer Stress eine Tendenz zur Aggregatbildung von Proteinen im allgemeinen und von Immunglobulinen im speziellen auslöst.

Nach dem erfindungsgemässen Verfahren ist eine stabile humane IgA-Lösung erhältlich, welche sich durch eine geringe Tendenz zur Aggregatbildung auszeichnet und zur pharmazeutischen Anwendung geeignet ist. Vorzugsweise wird das IgA an dem anorganischen Träger nicht adsorbiert, am meisten bevorzugt wird das IgA auch während weiterer Reinigungsschritte an keinem Trägermaterial adsorbiert.

Dieses IgA-Präparat ist so stabil, dass es ohne weiteres einer Hitzebehandlung oder Lyophilisierung unterzogen werden kann oder wesentlichee Aggregatsbildung.

Die erfindungsgemässe IgA-Lösung zeichnet sich, wie bereits erwähnt, durch eine geringe Tendenz zur Aggregatbildung aus.

Diese Tendenz zur Aggregatbildung kann beispielsweise mittels eines leicht durchzuführenden Tests bestimmt werden: Die zu untersuchende Präparation wird in einem physiologischen Medium als Lösung, gegebenenfalls nach Rekonstitution des Lyophilisats, in einer Konzentration von 2 mg IgA/ml bzw. 0,5 mg IgA/ml bei einer Temperatur von 63°C für 20 min inkubiert und dann die gebildete Menge an Aggregaten mittels hierfür gängiger Methoden, beispielsweise mittels elektrophoretischer Methoden oder mittels Gelfiltrationsanalyse bestimmt. Die erfindungsgemässe Lösung weist dann einen Aggregatgehalt von weniger als 50%, vorzugsweise weniger als 40%, mehr bevorzugt weniger als 30%, weiters bevorzugt weniger als 20%, noch mehr bevorzugt weniger als 10% und am meisten bevorzugt weniger als 5% auf.

Der Test kann auch bei anderen Temperaturen, vorzugsweise von mindestens 60°C, unter sonst gleichen Bedingungen, wie bereits beschrieben, durchgeführt werden, wobei dann die Zeit für das Inkubieren der zu untersuchenden Probe pro °C 4 min entsprechend zu verkürzen oder zu verlängern ist. Beispielsweise kann der Test bei einer Temperatur von 60°C für 32 min oder bei 65°C für 12 min durchgeführt werden.

Die gemäss dem erfindungsgemässen Verfahren hergestellte IgA-Lösung zeichnet sich durch die native Konformation des IgA aus. Dies ist besonders wichtig für biologische Funktionen, für die ein intaktes Immunglobulin Voraussetzung ist, wie dies beispielsweise durch Tests zur Bestimmung der Toxinneutralisation, Niedermodulation von Zytokinen bzw. Hemmung der Bildung von Sauerstoffradikalen bestimmbar ist.

Unter Aggregaten versteht man solche Molekülverbände, die ein Molekulargewicht grösser als 400 kD aufweisen. Hochmolekulare Aggregate weisen im allgemeinen ein Molekulargewicht von mehr als 600 kD auf. IgA-Monomere weisen definitionsgemäss ein Molekulargewicht im Bereich zwischen 150 und 180 kD auf. Das Molekulargewicht der IgA-Dimere liegt in einem Bereich zwischen 300 und 360 kD. Das IgA-Dimer kann unter Umständen auch die J-Kette bzw. die sekretorische Komponente enthalten.

Gemäss dem erfindungsgemässen Verfahren ist eine stabile IgA-Lösung erhältlich, die beispielsweise weniger als 10% Aggregate aufweist, vorzugsweise liegt der Aggregatanteil unter 8%, und am meisten bevorzugt weist die Lösung einen Aggregatanteil von weniger als 5% auf.

Der Gehalt an Dimeren in der erfindungsgemässen Lösung beträgt beispielsweise bis zu 10%, vorzugsweise bis zu 8%, am meisten bevorzugt enthält die Lösung 1 bis 2% Dimere.

Der Gehalt an IgA-Monomeren kann zwischen 80 und 99% liegen, vorzugsweise liegt der Anteil an monomerem IgA um 90%.

Die durch das erfindungsgemässe Verfahren erhaltene IgA-Lösung ist für die pharmazeutische Anwendung geeignet und lagerstabil. So ist die erfindungsgemässe IgA-Lösung beispielsweise bei 4°C bis zu 4 Jahre lagerstabil, vorzugsweise bis zu 2 Jahre, oder bei Raumtemperatur 1 Monat bis zu 2 Jahre, ohne wesentliche Aggregatbildung, d.h. weniger als 10% Aggregatgehalt in der Lösung.

Bei höheren Temperaturen, wie sie beispielsweise bei entsprechenden Verfahren zur Inaktivierung von gegebenenfalls in der Lösung vorhandenen Viren angewandt werden, also bei Temperaturen beispielsweise zwischen 50 und 80°C, vorzugsweise zwischen 60 und 65°C, ist die erfindungsgemässe Lösung ebenfalls stabil und weist eine geringe Tendenz zur Aggregatbildung auf. Diese verminderte Tendenz zur Aggregatbildung kann beispielsweise mittels des an früherer Stelle beschriebenen Testsystems festgestellt werden.

In der erfindungsgemäß erhaltenen IgA-Lösung liegen die Subklassen IgA1 und IgA2 vorzugsweise in einem Verhältnis vor, das der Zusammensetzung von nativem IgA entspricht. Vorzugsweise handelt es sich um polyvalentes IgA.

Das IgA-Präparat kann zusammen mit üblichen pharmazeutischen Träger- und/oder Verdünnungsmitteln kombiniert werden. Es kann in flüssiger oder lyophilisierter Form vorliegen und ist lagerstabil.

Pharmazeutische Zusammensetzungen, die das erfindungsgemäss gewonnene IgA enthalten, können neben diesem auch andere aktive Bestandteile (Wirksubstanzen) umfassen, sofern diese mit IgA kompatibel und für die Zweckbestimmung der pharmazeutischen Zusammenstzung geeignet und zweckmässig sind.

Die Herstellung der pharmazeutischen Zusammensetzungen erfolgt nach bekannten Verfahren und richtet sich insbesondere nach der Art der beabsichtigten Darreichungsform. Die Verabreichung der erfindungsgemäss gewonnenen IgA-Präparate kann auf lokalem, mukosalem, z.B. oralem, oder systemischen Weg, erfolgen.

Die das erfindungsgemäss gewonnene IgA-Präparat enthaltenden pharmazeutischen Zusammensetzungen sind insbesondere zur Vorbeugung und Behandlung von Entzündungen, Infektionen und/oder Allergien geeignet. Die Dosierung hängt vom Verabreichungsweg und der Häufigkeit der Verabreichung sowie vom Ausmass und der Schwere der Erkrankung ab. Wenn hohe Gesamtdosen an IgA zu verabreichen sind, so ist es häufig bevorzugt, IgA in mehreren kleinen Dosierungsmengen während es Tages verteilt zu verabreichen. IgA kann z.B. oral (normalerweise 1 bis 10 g/Tag oder in schweren Fällen mehr) vorzugsweise in 3 oder mehr Dosen verabreicht werden. Die Dosierung richtet sich aber insbesondere auch nach dem Allgemeinzustand und Alter des Patienten und nach der Schwere der Erkrankung.

Besonders bevorzugt ist die Verabreichung von IgA auf systemischen Wegen, z.B. mittels intravenöser Injektionen, kontinuierlicher Infusionen oder beidem. Typischerweise werden 50 bis 2000 mg IgA/kg/Tag verabreicht. In seltenen Fällen kann die Verabreichung auch intramuskulär erfolgen, normalerweise mit einer Dosierung von 50 bis 100 mg IgA/kg/Tag.

Ausserdem kann das erfindungsgemäss gewonnene IgA auch mukosal verabreicht werden, z.B. mittels Inhalationen (bis zu 10 ml/Tag, 10 bis 100 mg IgA/ml), nasal (15 bis 200 mg/ml) mit Hilfe von Sprays oder Tropfen, oder lokal durch intraartikuläre Injektionen (die nach Bedarf 1 bis 5 ml einer Lösung von 10 bis 100 mg IgA/ml enthalten). Andere Verabreichungswege umfassen Suppositorien (100 bis 1000 mg IgA/Dosis) und transdermale Pflaster. Transdermale Pflaster eignen sich besonders, um Hautentzündungen zu behandeln.

Als Darreichungsformen kommen insbesondere solche infrage, die für die Vorbeugung und Behandlung von Virusinfektionen üblich sind, insbesondere orale Darreichungsformen, wie z.B. Kapseln, Tabletten, Granulate, Pellets, Mini-Pellets und Mikrokapseln. Bei der Behandlung von viralen Darminfektionen ist es bevorzugt, die Granulate bzw. Mini-Pellets, etc. mit einem dafür üblichen magensaftresistenten, darmlöslichen Überzug zu versehen.

Die in den beiliegenden Abbildungen gezeigten Elutionsdiagramme wurden durch Gelfiltrationsanalyse der jeweiligen Zwischenstufen bzw. Endstufe erhalten. Die Abbildungen stellen folgendes dar:
- Abb. 1: Gelfiltrationsanalyse des Startmaterials;
- Abb. 2: Gelfiltrationsanalyse des nach Fractogel TMAE erhaltenen Materials gemäss Beispiel 1;
- Ab. 3: Gelfiltrationsanalyse des nach Hydroxylapatit-Behandlung erhaltenen Materials gemäss Beispiel 1;
- Abb. 4: Gelfiltrationsanalyse des nach Ammoniumsulfat-Fällung erhaltenen Materials gemäss Beispiel 1.

Der Pfeil bezeichnet jeweils den IgA enthaltenden Peak.

Die nachfolgenden Beispiele sollen die Erfindung näher erläutern, ohne sie jedoch darauf zu beschränken.

### Beispiel 1

Wie in EP-506 651 beschrieben, wurde aus humanem Plasma eine Cohn II+III-Fraktion hergestellt, die mit einem Phosphat-Acetat-Puffer extrahiert wurde. Dieses Material wurde in Ethanol bei einem pH-Wert von 5,3 und einer Temperatur von -- -2°C auf eine Konzentration von 12% versetzt, wobei sich ein Niederschlag bildete, der abgetrennt wurde. Die erhaltene Paste wurde extrahiert und der Extrakt zur Abtrennung von Plasminogen mit Lysin-Agarose behandelt. Das nicht gebundene Material wurde erfindungsgemäss weiterbehandelt:

Der Lysin-Agarose-Überstand wurde 60 min bei 18900 x g bei 4°C zentrifugiert, um die unlöslichen Bestandteile abzutrennen. Der Niederschlag wurde verworfen. Der Überstand wurde bei Raumtemperatur auf einem Amicon Spiralmodul S1Y30 Cross Flow gegen das dreifache Probenvolumen von 50 mM Natriumacetat/Essigsäure-Puffer, pH 5,0, dialysiert und anschliessend 15 min bei 18900 x g bei 4°C zentrifugiert.

Das dialysierte Material wurde mit einem Anionenaustauscher im Batch-Verfahren versetzt. Dazu wurde die Probe mit dem gleichen Volumen Ionenaustauscher (Fractogel EMD TMAE 650(M), Korngrösse 0,04 bis 0,09 mm; Merck, Darmstadt, DE; 1:2 suspendiert in 50 mM Natriumacetat/Essigsäure-Puffer, pH 5,0) versetzt, so dass pro 20 mg Protein 2 ml Gel-Suspension vorlagen. Die Suspension wurde über Nacht bei 4°C gerührt. Nicht gebundenes Material wurde über eine Nutsche abgetrennt und das Gel zweimal mit 50 mM Natriumacetat/Essigsäure-Puffer, pH 5,0, gewaschen. Anschliessend wurde das Gel 2 h bei 4°C mit 50 mM Natriumacetat/Essigsäure-Puffer + 0,5 M NaCl, pH 5,5 gerührt. Dann wurde das Gel über eine Nutsche von den mit diesem Puffer eluierten Proteinen abgetrennt. Diese Elution wurde zweimal durchgeführt.

Das eluierte Material wurde gegen Puffer A (PBS, pH 7,4) dialysiert. Dem Retentat wurden 3% Puffer B (0,5 M NaH₂PO₄/Na₂HPO₄ + 150 mM NaCl, pH 6,8) zugesetzt. Dieses Material wurde mit Hydroxylapatit (BioRad, Richmond, CA/USA; Macro Prep Ceramic Hydroxylapatit; 20 Micron) im Batch-Verfahren versetzt. Dazu wurde der Hydroxylapatit in einer Mischung aus 97% Puffer A (PBS, pH 7,4 und 3% Puffer B (0,5 M NaH₂PO₄/Na₂HPO₄ + 150 mM NaCl, pH 6,8) äquilibriert und im gleichen Puffergemisch suspendiert. Pro 2 mg IgA wurde 1 ml Hydroxylapatit eingesetzt. Der Ansatz wurde über Nacht bei 4°C gerührt. Der Überstand wurde abgenutscht und der Hydroxylapatit bei 4°C mit einer Mischung aus 97% Puffer A (PBS, pH 7,4) und 3% Puffer B (0,5 M NaH₂PO₄/Na₂HPO₄ + 150 mM NaCl, pH 6,8) gewaschen. Der Waschüberstand wurde abgenutscht und mit dem ersten Überstand vereint.

Die Eluate des Hydroxylapatit-Batch-Verfahrens wurden unmittelbar oder nach Ankonzentrierung unter Rühren mit Ammoniumsulfat bis zu einer Konzentration von 1,8 M versetzt. Daraufhin wurde noch 1 h bei Raumtemperatur gerührt und anschliessend das gebildete Präzipitat 15 min bei 2410 x g abzentrifugiert. Der Niederschlag wurde in PBS, pH 7,4, resuspendiert und gegen destilliertes Wasser dialysiert (Dialyseschlauch von Spectra/Por, Nr. 2, MWCO 12-14.000). Das während der Dialyse entstandene Präzipitat wurde abzentrifugiert und der Überstand steril filtriert.

Das so hergestellte Produkt wurde analysiert:
Die Analyse über Gelfiltration wurde auf einer analytischen Superdex 200 HR 10/30 FPLC-Säule (Pharmacia-LKB) mit PBS als Laufpuffer und einer Fliessgeschwindigkeit von 0,5 ml/min auf einer FPLC-Anlage (Pharmacia-LKB) durchgeführt. Die optische Dichte (OD) wurde bei 280 nm in einer Durchflussküvette gemessen und gegen das Elutionsvolumen (ml) aufgezeichnet.

IgA und IgG wurden mittels Radialimmundiffusion (Mancini G et al, Immunochemistry 2 (1965) 253-254) und der Gesamtproteingehalt nach der Methode von Lowry O H et al (J Biol Chem 193 (1951) 265-275) bestimmt.

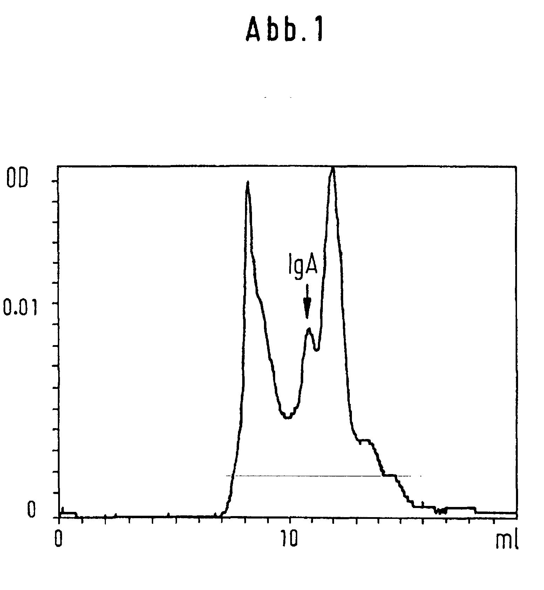

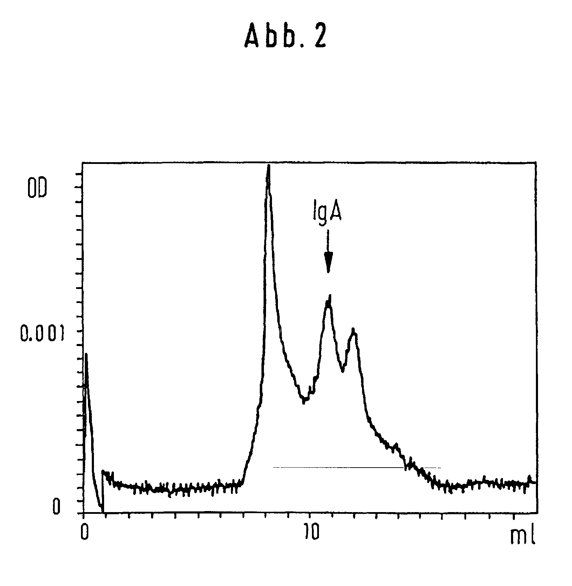

Es zeigte sich, dass die Ionenaustauscher-Behandlung zwar eine teilweise Anreicherung von IgA bewirkte (siehe Tabelle 1), dass aber der Anteil an IgG, hochmolekularen Proteinen und/oder Aggregaten weiterhin vorhanden war (siehe Abb. 1 und 2).

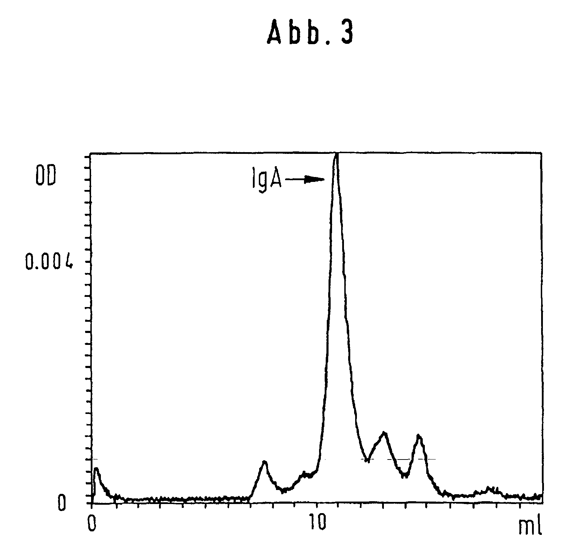

Die Hydroxylapatit Behandlung steigerte das Verhältnis von IgA zu IgG deutlich (siehe Tabelle 1 und Abb. 3). Das bedeutet, dass durch diese Behandlung IgA von IgG getrennt wurde.

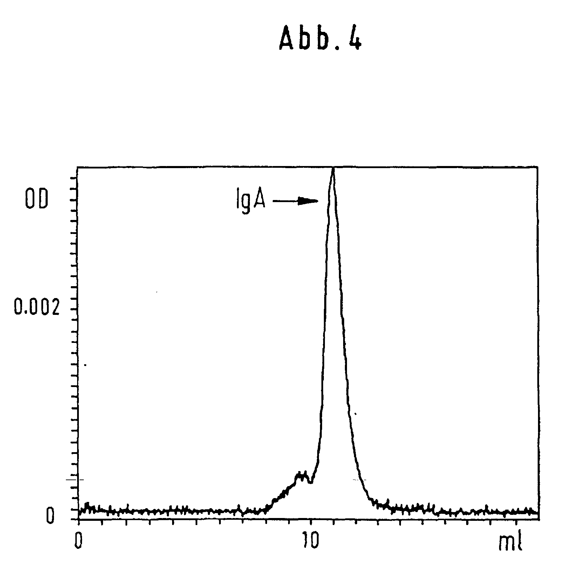

Das Endprodukt bestand grösstenteils aus IgA-Monomeren sowie einem geringen Anteil von IgA-Dimeren, die sich in Lösung aus IgA-Monomeren bildeten (siehe Tabelle 1 und Abb. 4).

**Tabelle 1**

| Behandlung | Gesamtprotein mg | IgA mg | IgG-Anteil mg | Verhältnis IgA/ Protein in % | Verhältnis IgA:IgG |
|---|---|---|---|---|---|
| Startmaterial | 5745 | 1084 | 770 | 19 | 1,4:1 |
| Fractogel | 1987 | 591 | 110 | 30 | 5,4:1 |
| Hydroxylapatit | 305 | 199 | 14 | 65 | 14,2:1 |
| Ammoniumsulfat | 132 | 120 | 5 | 91 | 24,0:1 |

## Patentansprüche

1. Verfahren zur Trennung von IgG und IgA in einem Immunglobulin-haltigen Ausgangsmaterial, **dadurch gekennzeichnet, dass**
i) IgG unter Bedingungen an das Trägermaterial Hydroxylapatit adsorbiert wird, unter denen IgA nicht daran adbsorbiert wird,
ii) IgA, das nicht an das Trägermaterial Hydroxylapatit adsorbiert wird, gewonnen wird, während IgG am Trägermaterial verbleibt.

2. Verfahren nach Anspruch 1, **dadurch gekennzeichnet, dass** IgG aus dem Absorbat gewonnen wird.

3. Verfahren nach einem der Ansprüche 1 und 2, **dadurch gekennzeichnet, dass** an das Hydroxylapatit hochmolekulare Proteine und/oder Proteinaggregate adsorbiert werden.

4. Verfahren nach Anspruch 1 bis 3, **dadurch gekennzeichnet, dass** das IgA bzw. das IgG durch weitere chromatographische Verfahren gereinigt wird.

5. Verfahren nach Anspruch 4, **dadurch gekennzeichnet, dass** das IgA bzw. das IgG durch Ionenaustauschchromatographie und/oder eine Fällung weiter gereinigt wird.

6. Verfahren nach einem oder mehreren der Ansprüche 1 bis 5, **dadurch gekennzeichnet, dass** ein Schritt zur Inaktivierung von Viren durchgeführt wird.

7. Verfahren nach einem oder mehreren der Ansprüche 1 bis 6, **dadurch gekennzeichnet, dass** IgA im wesentlichen frei von IgG erhalten wird.

8. Verfahren nach einem oder mehreren der Ansprüche 1 bis 7, **dadurch gekennzeichnet, dass** das Immunglobulin-haltige Ausgangsmaterial Serum, Plasma oder eine Plasmafraktion ist.

9. Verfahren nach einem oder mehreren der Ansprüche 1 bis 8, **dadurch gekennzeichnet, dass** das Immunglobulin-haltige Ausgangsmaterial ein mukosales Sekret ist.

10. Verfahren nach einem oder mehreren der Ansprüche 1 bis 9, **dadurch gekennzeichnet, dass** das Immunglobulin-haltige Ausgangsmaterial eine Cohn II+III-Fraktion ist.

## Claims

1. Process for separating IgG and IgA in an immunoglobulin-containing starting material, **characterised in that**
i) IgG is adsorbed on the carrier material hydroxylapatite under conditions, under which IgA is not adsorbed thereon,
ii) IgA, which is not adsorbed on the carrier material hydroxylapatite, is recovered, while IgG remains on the carrier material.

2. Process according to claim 1, **characterised in that** IgG is recovered from the adsorbate.

3. Process according to one of claims 1 and 2, **characterised in that** high-molecular proteins and/or protein aggregates are adsorbed on the hydroxylapatite.

4. Process according to claim 1 to 3, **characterised in that** the IgA or the IgG is purified by further chromatographic processes.

5. Process according to claim 4, **characterised in that** the IgA or the IgG is further purified by ion-exchange chromatography and/or precipitation.

6. Process according to one or more of claims 1 to 5, **characterised in that** a step for inactivating viruses is carried out

7. Process according to one or more of claims 1 to 6, **characterised in that** IgA is obtained essentially free of IgG.

8. Process according to one or more of claims 1 to 7, **characterised in that** the immunoglobulin-containing starting material is serum, plasma or a plasma fraction.

9. Process according to one or more of claims 1 to 8, **characterised in that** the immunoglobulin-containing starting material is a mucosal secretion.

10. Process according to one or more of claims 1 to 9, **characterised in that** the immunoglobulin-containing starting material is a Cohn II+III fraction.

## Revendications

1. Procédé d'isolement des immunoglobulines G et A dans un produit de départ contenant des immunoglobulines, **caractérisé en ce que**
(i) l'immunoglobuline G est adsorbée sur le porteur fixe hydroxylapatite, dans des conditions dans lesquelles l'immunoglobuline A n'y est pas adsorbée,
(ii) l'immunoglobuline A qui n'est pas adsorbée sur le porteur hydroxylapatite est extraite, alors que l'immunoglobuline G reste sur le porteur.

2. Procédé selon la revendication 1, **caractérisé en ce que** de l'immunoglobuline G est extraite du produit absorbé.

3. Procédé selon l'une quelconque des revendications 1 et 2, **caractérisé en ce que** des protéines polymoléculaires et/ou des agrégats de protéines sont adsorbés sur l'hydroxylapatite.

4. Procédé selon l'une quelconque des revendications 1 à 3, **caractérisé en ce que** l'immunoglobuline A ou l'immunoglobuline G est purifiée par des procédés de chromatographie ultérieurs.

5. Procédé selon la revendication 4, **caractérisé en ce que** l'immunoglobuline A ou l'immunoglobuline G est encore purifiée par chromatographie par échange d'ions et/ou par une précipitation.

6. Procédé selon l'une ou plusieurs des revendications 1 à 5, **caractérisé en ce qu'**une étape pour l'inactivation de virus est réalisée.

7. Procédé selon l'une quelconque des revendications 1 à 6, **caractérisé en ce que** l'immunoglobuline A obtenue est pratiquement exempte d'immunoglobuline G.

8. Procédé selon l'une ou plusieurs des revendications 1 à 7, **caractérisé en ce que** le produit de départ contenant de l'immunoglobuline est du sérum, du plasma ou une fraction de plasma.

9. Procédé selon l'une quelconque des revendications 1 à 8, **caractérisé en ce que** le produit de départ contenant de l'immunoglobuline est une sécrétion des muqueuses.

10. Procédé selon l'une ou plusieurs des revendications 1 à 9, **caractérisé en ce que** le produit de départ contenant de l'immunoglobuline est une fraction de II+III selon Cohn.
